# EUROPEAN PATENT SPECIFICATION

(11) **EP 3 398 589 B1**
(45) Date of publication and mention of the grant of the patent: **27.01.2021**
(21) Application number: 16882102.3
(22) Date of filing: 28.12.2016
(51) Int. Cl.: A61K 9/20, A61K 9/24, A61K 31/4184, A61K 31/4422

(54) **PHARMACEUTICAL COMPOSITE PREPARATION**
PHARMAZEUTISCHES KOMBINATIONSPRÄPARAT
PRÉPARATION COMPOSITE PHARMACEUTIQUE

(30) Priority: 28.12.2015 KR 20150187679
(43) Date of publication of application: 07.11.2018
(73) Proprietor: Shin Poong Pharmaceutical Co., Ltd., Ansan-si, Gyeonggi-do 15610 (KR)
(72) Inventor: RYU, Jei-Man, Gunpo-si Gyeonggi-do 15866 (KR); PARK, Woo-Ile, Anyang-si Gyeonggi-do 14074 (KR); KANG, Kyoung-Hwan, Seoul 08520 (KR); KIM, Woo-Kyung, Suwon-si Gyeonggi-do 16369 (KR); CHAE, Ah-Reum, Seongnam-si Gyeonggi-do 13626 (KR); KIM, Soo-Won, Anyang-si Gyeonggi-do 14054 (KR); JUNG, Hyun-Woo, Seoul 04411 (KR); LEE, Jae-Young, Yongin-si Gyeonggi-do 16843 (KR)
(74) Representative: Rückerl, Florian
(86) International application number: PCT/KR2016/015439
(87) International publication number: WO 2017/116150

(56) References cited:
- EP-A1- 2 165 702
- WO-A1-2008/044862
- WO-A1-2014/097209
- CN-A- 102 688 236
- KR-A- 20070 085 801
- KR-A- 20100 048 137
- KR-A- 20110 085 307
- KR-B1- 101 168 136
- KR-B1- 101 302 883
- US-A1- 2005 037 068
- RAO SHASHA ET AL: "Perspective and potential of oral lipid-based delivery to optimize pharmacological therapies against cardiovascular diseases", JOURNAL OF CONTROLLED RELEASE, ELSEVIER, AMSTERDAM, NL, vol. 193, 20 May 2014 (2014-05-20), pages 174-187, XP029084261, ISSN: 0168-3659, DOI: 10.1016/J.JCONREL.2014.05.013

## Description

### [TECHNICAL FIELD]

The present invention relates to a tablet comprising candesartan or candesartan cilexetil and amlodipine or its pharmaceutically acceptable salt as active ingredients, the tablet using a particular solubilizer in order to significantly improve the stability and dissolution properties of the active ingredients, said solubilzer being macrogol-15-hydroxystearate. In addition, the present invention relates to a method of preparing the tablet, said tablet comprising candesartan or candesartan cilexetil as the first active ingredient, amlodipine or its pharmaceutically acceptable salt as the second active ingredient and macrogol-15-hydroxystearate as a solubilizer.

### [BACKGROUND ART]

The number of patients suffering from hypertension would continuously increase all over the world, and reach to approximately 1.5 billion in the year 2025 according to several reports. The hypertension is one of the most common diseases, which occurs in about 30 percent or more of adults over the age thirty in Republic of Korea. It is reported that the hypertension develops various complications like vascular sclerosis, atherosclerosis, coronary artery disease, heart failure, stroke, etc.

In order to treat such a hypertension, non-pharmacological treatments like weight loss, exercise therapy, dietary therapy such as a low-salt diet, no smoking, reducing alcohol, etc. are suggested. However, it is generally difficult for most of the patients to adjust their blood pressures to the desired extent only by the non-pharmacological treatments which change their lifestyles and habits. For this reason, drug treatments are usually paired with non-pharmacological treatments. For the patients with stage 1 hypertension whose systolic blood pressure is 140 ∼ 159 mmHg or diastolic blood pressure is 90 ∼ 99 mmHg, it is recommended to begin the change of lifestyles and the drug treatments simultaneously. For the patients with stage 2 hypertension whose systolic blood pressure is 160 mmHg or more or diastolic blood pressure is 100 mmHg or more, it is recommended to administer at least two drugs together in order to lower blood pressures to the desired level as soon as possible and accordingly inhibit them from developing complications due to hypertension.

Drugs which have been developed for treating hypertension include thiazide diuretics, alpha blockers, beta blockers, alpha-beta blockers, angiotensin-converting enzyme (ACE) inhibitors, angiotensin II receptor blockers, calcium channel blockers, renin inhibitors, vasodilators, etc.

Said angiotensin II receptor blockers include, for example, candesartan having the following chemical structure:

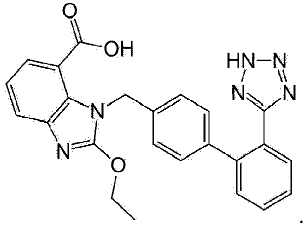

The candesartan has high efficacy in treating hypertension. However, due to its low water-solubility, there is a problem that the bioavailability in the body is not good when administered orally.

Thus, in order to increase solubility of candesartan compound and thereby improve bioavailability, candesartan cilexetil having the following chemical structure was developed:

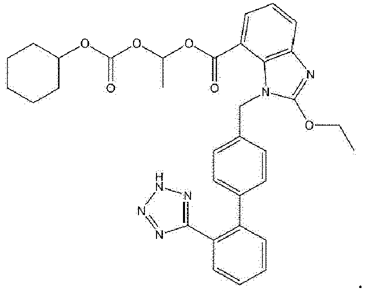

The candesartan cilexetil is marketed by AstraZeneca and Takeda Pharmaceuticals, under the trade names Blopress, Atacand, Amias, and Ratacand. It is a prodrug for increasing the bioavailability of candesartan compound. Nevertheless, there still remains a problem because the absolute bioavailability is about 40 % when the candesartan cilexetil is used as a solution, and the absolute bioavailability is about 15 % when the candesartan cilexetil is used as a tablet formulation.

Accordingly, various studies have been conducted in order to solve the problems shown in the candesartan or its prodrug form candesartan cilexetil.

For example, the international patent publication WO 2005/084648 discloses a pharmaceutical composition comprising candesartan cilexetil and water-soluble polymers such as polyvinyl alcohol, maltodextrin, xanthan gum, etc. The international patent publication WO 2010/060564 filed by BAYER discloses an active ingredient combination of candesartan cilexetil and nifedipine or nisoldipine as a calcium channel blocker. The international patent publications WO 2007/081232, WO 2010/150921, etc. disclose amlodipine as a calcium channel blocker for using in combination with candesartan.

However, it is reported that when candesartan or candesartan cilexetil is stored, it is degraded over time because of the interaction with other active ingredients or additives comprised in a combination formulation. For example, candesartan cilexetil shows a high stability against temperature, humidity and light when it exists solely in a solid state. On the contrary, various decomposition products such as desethyl candesartan are generated as impurities when candesartan cilexetil exists together with other active ingredients added in order to increase the bioavailability of candesartan cilexetil or obtain a synergistic effect in treating high blood pressure.

Therefore, when a pharmaceutical composition in the combination formulation comprising an active ingredient candesartan and other active ingredient(s) is stored in the long term, the increased level of impurities caused by the decomposition of candesartan cilexetil and/or other active ingredients has an adverse effect on the stability of pharmaceutical composition, and also lowers the efficacy of drug.

In addition, candesartan or candesartan cilexetil has a low water-solubility as mentioned above, and moreover its intrinsic dissolution may become even worse depending on the types and properties of other active ingredients or additives which are comprised in the combination formulation. If the dissolution of active ingredient in the gastrointestinal tract or in the body deteriorates, it is difficult to increase or maintain the level of drug in the blood within the desired time and to achieve the effect of treating or preventing a disease at the desired level.

### [TECHNICAL PROBLEM]

In the combination formulation comprising candesartan or candesartan cilexetil and amlodipine or its pharmaceutically acceptable salt as active ingredients, the inventors conducted various studies to improve the stability and the dissolution of tablet without counteracting the synergy effect of active ingredients.

Surprisingly, when a particular type of solubilizer, namely macrogol-15-hydroxystearate, is used in the combination formulation, it was experimentally proved that the decomposition of two active ingredients and the formation of impurities are markedly decreased, and the dissolution of active ingredients in the gastrointestinal tract or digestive tract of the body is optimized. The invention has been thus achieved.

### [TECHNICAL SOLUTION]

The present invention provides a tablet comprising candesartan or candesartan cilexetil as the first active ingredient, and amlodipine or its pharmaceutically acceptable salt as the second active ingredient, and comprising macrogol-15-hydroxystearate as a particular type of solubilizer.

The candesartan used in the present invention is a compound belonging to angiotensin II receptor blockers being a therapeutic agent of hypertension, and its chemical structure was described above.

The candesartan cilexetil used in the present invention is a prodrug of candesartan for increasing the solubility and the bioavailability of candesartan, and its chemical structure was also described above.

The amlodipine used in the present invention is a compound belonging to calcium channel blockers being a therapeutic agent of hypertension, angina, etc., and has the following chemical structure:

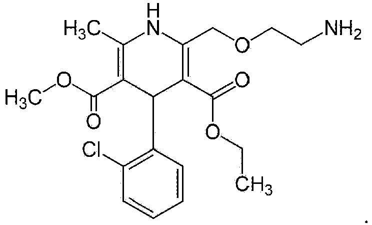

The pharmaceutically acceptable salt in the present invention includes acid addition salts or base addition salts which can be prepared by usual methods in the art. For example, it includes, but not limited to, acid addition salts formed from inorganic acids or organic acids such as acetic acid, aspartic acid, benzoic acid, besylic acid, carbonic acid, sulfuric acid, citric acid, fumaric acid, malic acid, phosphoric acid, etc., metal salts formed from reaction with alkali metal ions such as sodium, potassium, etc., or salts formed from reaction with ammonium ions.

The macrogol-15-hydroxystearate is a compound used as a solubilizer in the present invention, and its molecular formula is C₄₇H₉₄O₁₉ and its molecular weight is 963.23786 g/mol.

In one preferable embodiment of the present invention, the first active ingredient is candesartan cilexetil.

In one preferable embodiment of the present invention, the second active ingredient is amlodipine besylate.

The tablet according to the present invention can further comprise various additives such as plasticizers, excipients, disintegrating agents, fillers, weighting agents, binding agents, lubricants, colorants, wetting agents, sweetening agents, flavoring agents, preservatives, surfactants, diluents, antioxidants, etc. which can be commonly used in the art, if necessary. However, it should be noted that the tablet according to the present invention does not comprise any sugar alcohol (for example, mannitol, sorbitol, erythritol, etc.).

Optionally, the tablet according to the present invention can further comprise other active ingredients which are known in the art to have an effect of treating or preventing hypertension, for example thiazide diuretics, alpha blockers, beta blockers, alpha-beta blockers, angiotensin-converting enzyme (ACE) inhibitors, angiotensin II receptor blockers, calcium channel blockers, renin inhibitors, vasodilators, etc., provided that they do not counteract the synergy effect of combination formulation according to the present invention and do not cause side effects.

In one preferable embodiment of the present invention, the weight ratio of candesartan or candesartan cilexetil to macrogol-15-hydroxystearate is 1:0.03 ∼ 1:2. Preferably, the weight ratio is 1:0.05 ∼ 1:0.5, in particular 1:0.2. If the weight ratio is less than 1:0.03, there is a problem that the dissolution rate of tablet becomes noticeably worse. If the weight ratio is more than 1:2, the excessive agglomeration and coagulation of granules occurs, and thereby there is a problem that a tablet is hard to be formed, the tabletability is bad, and the dissolution rate and stability is decreased.

The tablet according to the present invention includes, but not limited to, compressed tablets, single-layer tablets, multi-layer tablets, nucleated tablets, sugar-coated tablets, film-coated tablets, gelatin-coated tablets, enteric-coated tablets, chewable tablets, fast-disintegrating tablets, etc. Preferably, the tablet is a single-layer tablet, a multi-layer tablet, or a nucleated tablet. Particularly, in terms of the stability and dissolution as demonstrated in the Examples below, the tablet is favorable to be a double-layer tablet.

In one preferable embodiment of the present invention, the tablet is a double-layer tablet comprising the first layer and the second layer, wherein the first layer comprises candesartan or candesartan cilexetil and macrogol-15-hydroxystearate, and the second layer comprises amlodipine or its pharmaceutically acceptable salt.

In one preferable embodiment of the present invention, the tablet according to the present invention is orally administered.

The dose and dosing interval of tablet according to the present invention can be properly selected considering various factors including age, sex, weight and medical condition of the patient, severity of disease to be treated or prevented (ex. hypertension), disease onset time, treatment period, etc. For example, an ordinarily skilled physician or pharmacist may determine the dose between about 1.0 mg /day to about 1.0 g/day. It may be administered once daily or in divided doses of two or more times daily. For example, when the tablet according to the present invention is administered to a patient who has suffered from hypertension once a day, it can be prepared by mixing the first active ingredient (candesartan or candesartan cilexetil) and the second active ingredient (amlodipine or its pharmaceutically acceptable salt) at a ratio such as 16 mg/10 mg, 16 mg/5 mg, 8 mg/5 mg, or 8 mg/2.5 mg.

The tablet according to the present invention can be stored for 6 weeks to 3 years. If it is kept at room temperature, it is possible to store for more than 3 years.

The method of preparing the tablet according to the present invention can be mainly classified into three, as follows.

In the first embodiment, the method of preparing the tablet according to the present invention comprises the steps:
a) preparing the first granules comprising the first active ingredient and a solubilizer;
b) adding a silicified microcrystalline cellulose and a lubricant to the first granules obtained in the step a) to prepare the first lubricated mixture;
c) preparing the second granules comprising the second active ingredient;
d) adding a lubricant to the second granules obtained in the step c) to prepare the second lubricated mixture; and
e) tableting the first lubricated mixture and the second lubricated mixture to prepare a double-layer tablet.

In the second embodiment, the method of preparing the tablet according to the present invention comprises the steps:
a) preparing the first granules comprising the first active ingredient, a solubilizer and a silicified microcrystalline cellulose;
b) adding a lubricant to the first granules obtained in the step a) to prepare the first lubricated mixture;
c) preparing the second granules comprising the second active ingredient;
d) adding a lubricant to the second granules obtained in the step c) to prepare the second lubricated mixture; and
e) tableting the first lubricated mixture and the second lubricated mixture to prepare a double-layer tablet.

In the third embodiment, the method of preparing the tablet according to the present invention comprises the steps:
a) preparing the first granules comprising the first active ingredient and a solubilizer;
b) preparing the second granules comprising the second active ingredient;
c) adding a silicified microcrystalline cellulose and a lubricant to the first granules and the second granules to prepare a lubricated mixture; and
d) tableting the lubricated mixture to prepare a single-layer tablet.

As used in the description of the invention and the claims, the singular forms "a", "an" and "the" are intended to include the plural forms as well, unless the context clearly indicates otherwise. And also, the term "and/or" as used herein refers to and encompasses any and all possible combinations of one or more of the associated listed constituents.

### [ADVANTAGEOUS EFFECTS]

The tablet according to the present invention has a combination formulation comprising candesartan or candesartan cilexetil as the first active ingredient, and amlodipine or its pharmaceutically acceptable salt as the second active ingredient.

In this case, by using macrogol-15-hydroxystearate as a particular type of solubilizer, the decomposition of two active ingredients for a long term is inhibited as demonstrated in the Examples below, and the formation of impurities is also minimized. Accordingly, the stability of drug is significantly increased. As a result, the tablet according to the present invention can be intactly kept without lowering the efficacy of active ingredients even though it is stored over the long period.

In addition, the water-solubility and bioavailability of the active ingredient candesartan is low, but its property dissolved from the tablet in the gastrointestinal tract or digestive tract of the body is remarkably improved in the tablet according to the present invention. Accordingly, the level of drug in the blood can be maintained at the constant level, and thus the effect to treat or prevent diseases such as hypertension can be achieved at the desired level.

Furthermore, the tablet according to the present invention has a good tabletability and productivity, and thus it is appropriate for the production of large scale in the form of tablet of the combination formulation which shows the good efficacy to treat or prevent hypertension, and the good stability and dissolution of active ingredients.

### [MODE FOR INVENTION]

The features of the invention will be explained below in more detail by reference to Examples.

### Example 1: Preparation of double-layer tablet

Hydroxypropylcellulose 6 g and macrogol-15-hydroxystearate 3 g were dissolved in purified water and ethanol to prepare a binding solution (1). Candesartan cilexetil 16 g, microcrystalline cellulose 65 g and pregelatinized starch 10 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Silicified microcrystalline cellulose 79 g was added to the screened granules (1), and mixed in a double-cone mixer. Then, magnesium stearate 1 g was added to obtain a lubricated mixture (1).

Hydroxypropylcellulose 5 g was dissolved in purified water and ethanol to prepare a binding solution (2). Amlodipine besylate 13.87 g, microcrystalline cellulose 71.13 g and pregelatinized starch 10 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Microcrystalline cellulose 64 g and pregelatinized starch 15 g were added to the screened granules (2), and mixed in a double-cone mixer. Then, magnesium stearate 1 g was added to obtain a lubricated mixture (2).

The lubricated mixture (1) and the lubricated mixture (2) were compressed into a double-layer tablet by a tableting machine (Autotab-200TR, Ichihashi seiki) using a 9.4 mm-diameter punch (the first layer weight: 180 g, the second layer weight: 180 g).

The obtained double-layer tablet was coated with Opadry (10.0 g/T) by a coating machine (Hi-coater, Freund).

### Example 2: Preparation of double-layer tablet

Hydroxypropylcellulose 6 g and macrogol-15-hydroxystearate 3 g were dissolved in purified water and ethanol to prepare a binding solution (1). Candesartan cilexetil 16 g, microcrystalline cellulose 65 g and pregelatinized starch 10 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Silicified microcrystalline cellulose 79 g was added to the screened granules (1), and mixed in a double-cone mixer. Then, magnesium stearate 1 g was added to obtain a lubricated mixture (1).

Hydroxypropylcellulose 2.5 g was dissolved in purified water and ethanol to prepare a binding solution (2). Amlodipine besylate 6.935 g, microcrystalline cellulose 35.565 g and pregelatinized starch 5 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Microcrystalline cellulose 32 g and pregelatinized starch 7.5 g were added to the screened granules (2), and mixed in a double-cone mixer. Then, magnesium stearate 0.5 g was added to obtain a lubricated mixture (2).

The lubricated mixture (1) and the lubricated mixture (2) were compressed into a double-layer tablet by a tableting machine (Autotab-200TR, Ichihashi seiki) using an 8.5 mm-diameter punch (the first layer weight: 180 g, the second layer weight: 90 g).

The obtained double-layer tablet was coated with Opadry (7.5 g/T) by a coating machine (Hi-coater, Freund).

### Example 3: Preparation of double-layer tablet

Hydroxypropylcellulose 3 g and macrogol-15-hydroxystearate 1.5 g were dissolved in purified water and ethanol to prepare a binding solution (1). Candesartan cilexetil 8 g, microcrystalline cellulose 32.5 g and pregelatinized starch 5 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Silicified microcrystalline cellulose 39.5 g was added to the screened granules (1), and mixed in a double-cone mixer. Then, magnesium stearate 0.5 g was added to obtain a lubricated mixture (1).

Hydroxypropylcellulose 2.5 g was dissolved in purified water and ethanol to prepare a binding solution (2). Amlodipine besylate 6.935 g, microcrystalline cellulose 35.565 g and pregelatinized starch 5 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Microcrystalline cellulose 32 g and pregelatinized starch 7.5 g were added to the screened granules (2), and mixed in a double-cone mixer. Then, magnesium stearate 0.5 g was added to obtain a lubricated mixture (2).

The lubricated mixture (1) and the lubricated mixture (2) were compressed into a double-layer tablet by a tableting machine (Autotab-200TR, Ichihashi seiki) using a 7.5 mm-diameter punch (the first layer weight: 90 g, the second layer weight: 90 g).

The obtained double-layer tablet was coated with Opadry (5.0 g/T) by a coating machine (Hi-coater, Freund).

### Example 4: Preparation of double-layer tablet

Hydroxypropylcellulose 3 g and macrogol-15-hydroxystearate 1.5 g were dissolved in purified water and ethanol to prepare a binding solution (1). Candesartan cilexetil 8 g, microcrystalline cellulose 32.5 g and pregelatinized starch 5 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Silicified microcrystalline cellulose 39.5 g was added to the screened granules (1), and mixed in a double-cone mixer. Then, magnesium stearate 0.5 g was added to obtain a lubricated mixture (1).

Hydroxypropylcellulose 2.5 g was dissolved in purified water and ethanol to prepare a binding solution (2). Amlodipine besylate 3.468 g, microcrystalline cellulose 39.032 g and pregelatinized starch 5 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Microcrystalline cellulose 32 g and pregelatinized starch 7.5 g were added to the screened granules (2), and mixed in a double-cone mixer. Then, magnesium stearate 0.5 g was added to obtain a lubricated mixture (2).

The lubricated mixture (1) and the lubricated mixture (2) were compressed into a double-layer tablet by a tableting machine (Autotab-200TR, Ichihashi seiki) using a 7.5 mm-diameter punch (the first layer weight: 90 g, the second layer weight: 90 g).

The obtained double-layer tablet was coated with Opadry (5.0 g/T) by a coating machine (Hi-coater, Freund).

### Example 5: Preparation of single-layer tablet

Hydroxypropylcellulose 6 g and macrogol-15-hydroxystearate 3 g were dissolved in purified water and ethanol to prepare a binding solution (1). Candesartan cilexetil 16 g, microcrystalline cellulose 65 g and pregelatinized starch 10 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Hydroxypropylcellulose 5 g was dissolved in purified water and ethanol to prepare a binding solution (2). Amlodipine besylate 13.87 g, microcrystalline cellulose 71.13 g and pregelatinized starch 10 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Silicified microcrystalline cellulose 79 g, microcrystalline cellulose 64 g and pregelatinized starch 15 g were added to the screened granules (1) and the screened granules (2), and mixed in a double-cone mixer. Then, magnesium stearate 2 g was added to obtain a lubricated mixture.

The lubricated mixture was compressed into a single-layer tablet by a tableting machine (Autotab-200TR, Ichihashi seiki) using a 9.4 mm-diameter punch (tablet weight: 360 g/T).

The obtained single-layer tablet was coated with Opadry (10.0 g/T) by a coating machine (Hi-coater, Freund).

### Example 6: Preparation of double-layer tablet

Hydroxypropylcellulose 6 g and macrogol-15-hydroxystearate 3 g were dissolved in purified water and ethanol to prepare a binding solution (1). Candesartan cilexetil 16 g, microcrystalline cellulose 65 g, pregelatinized starch 10 g and silicified microcrystalline cellulose 79 g were uniformly mixed in a high speed mixer to prepare a mixture (1). The binding solution (1) was added onto the mixture (1), granulated, and then dried. Screened granules (1) were obtained by sieving with a 20-mesh sieve in semi-dried state, and sieving with a 30-mesh sieve in dried state.

Magnesium stearate 1 g was added to the screened granules (1) to obtain a lubricated mixture (1).

Hydroxypropylcellulose 5 g was dissolved in purified water and ethanol to prepare a binding solution (2). Amlodipine besylate 13.87 g, microcrystalline cellulose 135.13 g and pregelatinized starch 25 g were pre-mixed and mixed in a double-cone mixer to prepare a mixture (2). The binding solution (2) was added onto the mixture (2), granulated, and then dried. Screened granules (2) were obtained by sieving with a 20-mesh sieve in semi-dried state, and sieving with a 30-mesh sieve in dried state.

Magnesium stearate 1 g was added to the screened granules (2) to obtain a lubricated mixture (2).

The lubricated mixture (1) and the lubricated mixture (2) were compressed into a double-layer tablet by a tableting machine (Autotab-200TR, Ichihashi seiki) using a 9.4 mm-diameter punch (the first layer weight: 180 g, the second layer weight: 180 g).

The obtained double-layer tablet was coated with Opadry (10.0 g/T) by a coating machine (Hi-coater, Freund).

Table 1 below shows the composition and weight of tablets prepared in Examples 1 to 6. In Table 1, "Ex." stands for "Example". The product prepared in Ex. 1, 2, 3, 4 and 6 respectively is a double-layer tablet, whereas the product prepared in Ex. 5 is a single-layer tablet. In Table 1, "the extragranular mixing-part" indicates the part formed on the outside of granule by mixing additional materials following granulation.

**[Table 1]**

| Composition | Weight (g) | | | | | |
|---|---|---|---|---|---|---|
| | Ex. 1 | Ex. 2 | Ex. 3 | Ex. 4 | Ex. 5 | Ex. 6 |
| | | | | | | |

| **The granule-part in the first layer of double-layer tablet** | | | | | | |
|---|---|---|---|---|---|---|
| candesartan cilexetil | 16.00 | 16.00 | 8.00 | 8.00 | - | 16.00 |
| microcrystalline cellulose | 65.00 | 65.00 | 32.50 | 32.50 | - | 65.00 |
| pregelatinized starch | 10.00 | 10.00 | 5.00 | 5.00 | - | 10.00 |
| hydroxypropylcellulose | 6.00 | 6.00 | 3.00 | 3.00 | - | 6.00 |
| macrogol-15-hydroxystearate | 3.00 | 3.00 | 1.50 | 1.50 | - | 3.00 |
| silicified microcrystalline cellulose | - | - | - | - | - | 79.00 |
| | | | | | | |

| **The extragranular mixing-part in the first layer of double-layer tablet** | | | | | | |
|---|---|---|---|---|---|---|
| silicified microcrystalline cellulose | 79.00 | 79.00 | 39.50 | 39.50 | - | - |
| magnesium stearate | 1.00 | 1.00 | 0.50 | 0.50 | - | 1.00 |
| | | | | | | |

| **The granule-part in the second layer of double-laver tablet** | | | | | | |
|---|---|---|---|---|---|---|
| amlodipine besylate | 13.87 | 6.935 | 6.935 | 3.468 | - | 13.87 |
| microcrystalline cellulose | 71.13 | 35.565 | 35.565 | 39.032 | - | 135.13 |
| pregelatinized starch | 10.00 | 5.00 | 5.00 | 5.00 | - | 25.00 |
| hydroxypropylcellulose | 5.00 | 2.50 | 2.50 | 2.50 | - | 5.00 |
| | | | | | | |

| **The extragranular mixing-part in the second layer of double-layer tablet** | | | | | | |
|---|---|---|---|---|---|---|
| microcrystalline cellulose | 64.00 | 32.00 | 32.00 | 32.00 | - | - |
| pregelatinized starch | 15.00 | 7.50 | 7.50 | 7.50 | - | - |
| magnesium stearate | 1.00 | 0.50 | 0.50 | 0.50 | - | 1.00 |
| sugar alcohol (ex. mannitol) | - | - | - | - | - | - |

| | | | | | | |
|---|---|---|---|---|---|---|
| **The first-granule-part in the single-layer tablet** | | | | | | |
| candesartan cilexetil | - | - | - | - | 16.00 | - |
| microcrystalline cellulose | - | - | - | - | 65.00 | - |
| pregelatinized starch | - | - | - | - | 10.00 | - |
| hydroxypropylcellulose | - | - | - | - | 6.00 | - |
| macrogol-15-hydroxystearate | - | - | - | - | 3.00 | - |
| | | | | | | |

| **The second-granule-part in the single-layer tablet** | | | | | | |
|---|---|---|---|---|---|---|
| amlodipine besylate | - | - | - | - | 13.87 | - |
| microcrystalline cellulose | - | - | - | - | 71.13 | - |
| pregelatinized starch | - | - | - | - | 10.00 | - |
| hydroxypropylcellulose | - | - | - | - | 5.00 | - |
| | | | | | | |

| **The extragranular mixing-part in the single-layer tablet** | | | | | | |
|---|---|---|---|---|---|---|
| microcrystalline cellulose | - | - | - | - | 64.00 | - |
| silicified microcrystalline cellulose | - | - | - | - | 79.00 | - |
| pregelatinized starch | - | - | - | - | 15.00 | - |
| magnesium stearate | - | - | - | - | 2.00 | - |
| | | | | | | |

| **The coating-part** | | | | | | |
|---|---|---|---|---|---|---|
| OPADRY | 10.00 | 7.50 | 5.00 | 5.00 | 10.00 | 10.00 |
| | | | | | | |
| **Total weight** | 370.0 | 277.5 | 185.0 | 185.0 | 370.0 | 370.0 |

### Comparative Example 1

A double-layer tablet was prepared by the same method as in Example 1, except that polyethylene glycol-6000 3 g was used instead of macrogol-15-hydroxystearate 3 g as a solubilizer.

### Comparative Example 2

A double-layer tablet was prepared by the same method as in Example 1, except that beta-cyclodextrin 3 g was used instead of macrogol-15-hydroxystearate 3 g as a solubilizer.

### Comparative Example 3

A double-layer tablet was prepared by the same method as in Example 1, except that d-α-tocopheryl polyethylene glycol-1000 succinate 3 g was used instead of macrogol-15-hydroxystearate 3 g as a solubilizer.

### Comparative Example 4

A double-layer tablet was prepared by the same method as in Example 1, except that sodium lauryl sulfate 3 g was used instead of macrogol-15-hydroxystearate 3 g as a solubilizer.

### Comparative Example 5

A double-layer tablet was prepared by the same method as in Example 1, except that microcrystalline cellulose 3 g was further added instead of using a solubilizer.

Table 2 below shows the composition and weight of tablets prepared in Example 1 and Comparative Examples 1 to 5. In Table 2, "Ex." stands for "Example", and "Comp. Ex." stands for "Comparative Example". The product prepared in Ex. 1 and Comp. Ex. 1 to 5 respectively is a double-layer tablet. In Table 2, "the extragranular mixing-part" indicates the part formed on the outside of granule by mixing additional materials following granulation.

**[Table 2]**

| Composition | Weight (g) | | | | | |
|---|---|---|---|---|---|---|
| | Ex. 1 | Comp. Ex. 1 | Comp. Ex. 2 | Comp. Ex. 3 | Comp. Ex. 4 | Comp. Ex. 5 |
| | | | | | | |

| **The granule-part in the first layer of double-layer tablet** | | | | | | |
|---|---|---|---|---|---|---|
| candesartan cilexetil | 16.00 | 16.00 | 16.00 | 16.00 | 16.00 | 16.00 |
| microcrystalline cellulose | 65.00 | 65.00 | 65.00 | 65.00 | 65.00 | 68.00 |
| pregelatinized starch | 10.00 | 10.00 | 10.00 | 10.00 | 10.00 | 10.00 |
| hydroxypropylcellulose | 6.00 | 6.00 | 6.00 | 6.00 | 6.00 | 6.00 |
| macrogol-15-hydroxystearate | 3.00 | - | - | - | - | - |
| polyethylene glycol-6000 | - | 3.00 | - | - | - | - |
| beta -cyclodextrin | | - | 3.00 | - | - | - |
| d-α-tocopheryl polyethylene glycol-1000 succinate | | - | - | 3.00 | - | - |
| sodium lauryl sulfate | - | - | - | - | 3.00 | - |
| | | | | | | |

| **The extragranular mixing-part in the first layer of double-layer tablet** | | | | | | |
|---|---|---|---|---|---|---|
| silicified microcrystalline cellulose | 79.00 | 79.00 | 79.00 | 79.00 | 79.00 | 79.00 |
| magnesium stearate | 1.00 | 1.00 | 1.00 | 1.00 | 1.00 | 1.00 |
| | | | | | | |

| **The granule-part in the second layer of double-laver tablet** | | | | | | |
|---|---|---|---|---|---|---|
| amlodipine besylate | 13.87 | 13.87 | 13.87 | 13.87 | 13.87 | 13.87 |
| microcrystalline cellulose | 71.13 | 71.13 | 71.13 | 71.13 | 71.13 | 71.13 |
| pregelatinized starch | 10.00 | 10.00 | 10.00 | 10.00 | 10.00 | 10.00 |
| hydroxypropylcellulose | 5.00 | 5.00 | 5.00 | 5.00 | 5.00 | 5.00 |
| | | | | | | |

| **The extragranular mixing-part in the second layer of double-layer tablet** | | | | | | |
|---|---|---|---|---|---|---|
| microcrystalline cellulose | 64.00 | 64.00 | 64.00 | 64.00 | 64.00 | 64.00 |
| pregelatinized starch | 15.00 | 15.00 | 15.00 | 15.00 | 15.00 | 15.00 |
| magnesium stearate | 1.00 | 1.00 | 1.00 | 1.00 | 1.00 | 1.00 |

| **The coating-part** | | | | | | |
|---|---|---|---|---|---|---|
| OPADRY | 10.00 | 10.00 | 10.00 | 10.00 | 10.00 | 10.00 |
| | | | | | | |
| **Total weight** | 370.0 | 370.0 | 370.0 | 370.0 | 370.0 | 370.0 |

### Experimental Example 1: Stability test

When the active ingredient candesartan is stored after prepared in the form of tablet together with other active ingredients or additives, it is often observed that it is decomposed as time passes. The typical impurity resulted from the decomposition of active ingredient candesartan includes desethyl candesartan, and any unknown impurities are also detected.

Similarly, amlodipine or its pharmaceutically acceptable salt, which is another active ingredient comprised in the tablet according to the present invention, results in degradation products by other active ingredients or additives present in the combination formulation. The typical impurity includes amlodipine impurity A (USP), and any unknown impurities are also detected. The chemical name of amlodipine impurity A (USP) is 3-ethyl 5-methyl 2-[(2-aminoethoxy)methyl]-4-(2-chlorophenyl)-6-methylpyridine-3,5-dicarboxylate fumarate.

It is general that a combination formulation such as the tablet according to the present invention shows the increased amount of impurities when it is stored in the long term. Thus, stability of tablets can be evaluated by determining the amount of impurities.

At first, the coated tablets prepared in Examples 1 to 6 and Comparative Examples 1 to 5 were separately put in a HDPE body/LDPE cap bottle. They were stored under the condition of 50°C/75% RH (relative humidity) for 6 weeks.

After that, twenty coated tablets were added into a 200 mL flask, filled up with water 20 mL, and mixed by shaking to completely disintegrate the tablets. Diluent A 95 mL was then added to the resultant, extracted by sonication while occasionally shaking, mixed with water 75 mL, and cooled at room temperature. And then, Diluent B was added to the resultant to make 200 mL of total solution volume. The obtained solution was filtered through a membrane filter to prepare a test solution.

20 µL of test solution and standard solution were subjected to the operating condition below, and the Liquid Chromatography of the General Test Methods in the Korean Pharmacopoeia was performed to calculate the peak areas A_{T} and A_{S} of the test solution and standard solution. Solvent peak and additives, benzenesulfonate derived peaks were excluded from the calculation. The additives derived peaks were excluded from the calculation by determining peaks of the placebo test solution prepared without main ingredients.

The impurities derived from candesartan cilexetil were measured at a wavelength of 215 nm, and the impurities derived from amlodipine besylate were measured at a wavelength of 238 nm. Any unknown impurities were measured at wavelengths 215 nm and 238 nm, respectively.
- Operating condition
   - Detector: Ultraviolet (UV)-absorption photometer (measurement wavelength: 215 nm and 238 nm)
   - Column: Waters Symmetry C8 (4.6 × 250 mm, 5 µm)
   - Column temperature: 30°C
   - Mobile phase A: triethylamine 4 mL added to water 1000 mL, then adjusted to a pH of 3.0 by phosphoric acid
   - Mobile phase B: a mixed solution of methanol and acetonitrile (10:90)
   - Diluent A: a mixed solution of methanol and acetonitrile (5:45)
   - Diluent B: a mixed solution of methanol, acetonitrile and water (5:45:50)
   - Flow rate: 1.0 mL/min

Table 3 below shows the gradient of mobile phase A and mobile phase B over time. The measurement of peak areas was performed at 36 min.

**[Table 3]**

| Time (min) | Mobile phase A (%, v/v) | Mobile phase B (%, v/v) |
|---|---|---|
| 0-3 | 65 | 35 |
| 3-18 | 65 →20 | 35 →80 |
| 18-23 | 20 | 80 |
| 23-28 | 20 →5 | 80 →95 |
| 28-36 | 5 | 95 |
| 36-37 | 5 →65 | 95 →35 |
| 37-47 | 65 | 35 |

Table 4 below shows the amount of known and/or unknown impurities derived from candesartan as a relative weight ratio based on 100% of candesartan content. Only the desethyl candesartan is representatively shown as the known impurity derived from candesartan.

**[Table 4]**

| | Condition | The representative known impurity derived from candesartan: desethyl candesartan | Total impurities derived from candesartan (including both known and unknown impurities) |
|---|---|---|---|
| | | 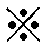 Acceptance criterion: not more than 2.0% | 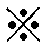 Acceptance criterion: not more than 4.7% |
| Ex. 1 | At the start of the test | 0.20 | 0.55 |
| | After stored at 50°C/ 75 % RH for 6 weeks | 0.58 | 1.76 |
| Ex. 2 | At the start of the test | 0.19 | 0.57 |
| | After stored at 50°C/75% RH for 6 weeks | 0.60 | 1.71 |
| Ex. 3 | At the start of the test | 0.18 | 0.56 |
| | After stored at 50°C/75% RH for 6 weeks | 0.55 | 1.70 |
| Ex. 4 | At the start of the test | 0.18 | 0.58 |
| | After stored at 50°C/75% RH for 6 weeks | 0.57 | 1.72 |
| Ex. 5 | At the start of the test | 0.20 | 0.54 |
| | After stored at 50°C/75% RH for 6 weeks | 0.75 | 2.22 |
| Ex. 6 | At the start of the test | 0.21 | 0.56 |
| | After stored at 50°C/75% RH for 6 weeks | 0.80 | 2.31 |
| Comp. Ex. 1 | At the start of the test | 0.21 | 0.60 |
| | After stored at 50°C/75% RH for 6 weeks | 1.24 | 3.41 |
| Comp. Ex. 2 | At the start of the test | 0.21 | 0.61 |
| | After stored at 50°C/75% RH for 6 weeks | 3.15 | 4.56 |
| Comp. Ex. 3 | At the start of the test | 0.21 | 0.66 |
| | After stored at 50°C/75% RH for 6 weeks | 2.12 | 3.78 |
| Comp. Ex. 4 | At the start of the test | 0.22 | 0.58 |
| | After stored at 50°C/75% RH for 6 weeks | 2.27 | 4.97 |
| Comp. Ex. 5 | At the start of the test | 0.24 | 0.67 |
| | After stored at 50°C/75% RH for 6 weeks | 3.38 | 5.23 |

As shown in Table 4, when the tablets of Comparative Examples 2, 3, 4 and 5 were stored under the condition of 50°C/75% RH for 6 weeks, the amount of known and/or unknown impurities derived from candesartan exceeded the acceptance criteria. In the tablet of Comparative Example 1, it was observed that the amount of known and/or unknown impurities derived from candesartan met the acceptance criteria, but was relatively higher than those in the tablet of Examples 1 to 6.

On the contrary, the amount of known and/or unknown impurities derived from candesartan in all the tablets of Examples 1 to 6 met the acceptance criteria. Particularly, in the double-layer tablet of Examples 1 to 4 which were prepared with extragranular mixing, the amount of impurities derived from candesartan active ingredient was remarkably low.

It means that candesartan or candesartan cilexetil among the active ingredients comprised in the combination formulation of tablet according to the present invention has the stability significantly improved by a particular type of solubilizer. Accordingly, although the tablet according to the present invention is stored for 6 weeks, it can be used in maintaining its efficacy intactly since the candesartan or candesartan cilexetil is almost not decomposed over time.

Table 5 below shows the total amount of unknown impurities derived from the combination formulation comprising candesartan and amlodipine as a relative weight ratio based on the content of combination formulation.

**[Table 5]**

| | Condition | The unknown impurities derived from the combination formulation of candesartan and amlodipine |
|---|---|---|
| | | 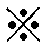 Acceptance criterion: not more than 0.2% |
| Ex. 1 | At the start of the test | 0.03 |
| | After stored at 50°C/75% RH for 6 weeks | 0.10 |
| Ex. 2 | At the start of the test | 0.03 |
| | After stored at 50°C/75% RH for 6 weeks | 0.11 |
| Ex. 3 | At the start of the test | 0.03 |
| | After stored at 50°C/75% RH for 6 weeks | 0.10 |
| Ex. 4 | At the start of the test | 0.03 |
| | After stored at 50°C/75% RH for 6 weeks | 0.10 |
| Ex. 5 | At the start of the test | 0.03 |
| | After stored at 50°C/75% RH for 6 weeks | 0.19 |
| Ex. 6 | At the start of the test | 0.03 |
| | After stored at 50°C/75% RH for 6 weeks | 0.18 |
| Comp. Ex. 1 | At the start of the test | 0.03 |
| | After stored at 50°C/75% RH for 6 weeks | 0.20 |
| Comp. Ex. 2 | At the start of the test | 0.02 |
| | After stored at 50°C/75% RH for 6 weeks | 0.23 |
| Comp. Ex. 3 | At the start of the test | 0.12 |
| | After stored at 50°C/75% RH for 6 weeks | 0.50 |
| Comp. Ex. 4 | At the start of the test | 0.02 |
| | After stored at 50°C/75% RH for 6 weeks | 0.25 |
| Comp. Ex. 5 | At the start of the test | 0.02 |
| | After stored at 50°C/75% RH for 6 weeks | 0.24 |

As shown in Table 5, when the tablets of Comparative Examples 1 to 5 are stored under the condition of 50°C/75% RH for 6 weeks, it was observed that the total amount of unknown impurities derived from the combination formulation comprising candesartan and amlodipine exceeds the acceptance criterion.

On the contrary, all the tablets of Examples 1 to 6 met the acceptance criterion. Particularly, in the double-layer tablet of Examples 1 to 4 which were prepared with extragranular mixing, the total amount of unknown impurities is remarkably low.

It means that when macrogol-15-hydroxystearate is used as a particular type of solubilizer, the stability of two active ingredients comprised in the combination formulation of tablet according to the present invention is significantly improved. Accordingly, although the tablet according to the present invention is stored for 6 weeks, it can be used in maintaining its efficacy intactly since all of two active ingredients are almost not decomposed over time.

### Experimental Example 2: Long-term stability test

The coated tablets prepared in Example 1 were put in a HDPE body/LDPE cap bottle, and were stored under the first condition of 25°C/60% RH for 12 months. In parallel with this, they were independently stored under the second condition of 40°C/70% RH for 6 months. After that, the amount of impurities was measured by the same method as in Experimental Example 1. The results thereof are shown in Tables 6 to 8 below.

**[Table 6]**

| | Condition | The representative known impurity derived from candesartan: desethyl candesartan | Total impurities derived from candesartan (including both known and unknown impurities) |
|---|---|---|---|
| | | 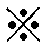 Acceptance criterion: not more than 2.0% | 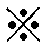 Acceptance criterion: not more than 4.7% |
| Ex. 1 | At the start of the test | 0.20 | 0.55 |
| | After stored at 25°C/60% RH for 12 months | 0.38 | 0.80 |
| | After stored at 40°C/70% RH for 6 months | 0.70 | 1.69 |

**Table 7]**

| | Condition | The representative known impurity derived from amlodipine: amlodipine impurity A (USP) | Total impurities derived from amlodipine (including both known and unknown impurities) |
|---|---|---|---|
| | | 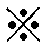 Acceptance criterion: not more than 0.5% | 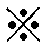 Acceptance criterion: not more than 0.7% |
| Ex. 1 | At the start of the test | 0.09 | 0.15 |
| | After stored at 25°C/60% RH for 12 months | 0.08 | 0.24 |
| | After stored at 40°C/70% RH for 6 months | 0.21 | 0.40 |

**[Table 8]**

| | Condition | The unknown impurities derived from the combination formulation of candesartan and amlodipine |
|---|---|---|
| | | 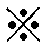 Acceptance criterion: not more than 0.2% |
| Ex. 1 | At the start of the test | 0.03 |
| | After stored at 25°C/60% RH for 12 months | 0.05 |
| | After stored at 40°C/70% RH for 6 months | 0.10 |

As shown in Tables 6 to 8, the tablet according to the present invention shows much less amount of impurities than the acceptance criteria when stored at 25°C/60% RH for 12 months, and also when stored at 40°C/70% RH for 6 months.

Therefore, the particular solubilizer used in the tablet of the present invention, macrogol-15-hydroxystearate inhibits the decomposition of two active ingredients comprised in the combination formulation, minimizes the formation of impurities, and significantly improves the storage stability even though the tablet is stored for a long term.

### Experimental Example 3: Dissolution test

The coated tablets prepared in Examples 1 to 6 and Comparative Examples 1 to 5 were evaluated by a dissolution test according to the paddle method in the Korean Pharmacopoeia at 37.0 ± 0.5°C and 75 rpm using 0.35% Polysorbate 20-containing phosphate buffer (pH 6.5) 900 mL.

10 mL of dissolution medium was taken, and then filtered through a membrane filter to prepare a test solution. Six tablets of Examples and Comparative Examples were tested respectively, and Table 9 below shows the average value, minimum value and maximum value of dissolution rate (%).
- Operating condition
   - Detector: Ultraviolet (UV)-absorption photometer (measurement wavelength: 238 nm)
   - Column: Waters Symmetry C18 (3.9 × 150 mm, 5 µm)
   - Column temperature: 30°C
   - Phosphate buffer (pH 6.5): 17.3 mL of 0.5 mol/L sodium hydrogen phosphate solution and water were added to 24 mL of 1 mol/L sodium dihydrogen phosphate solution to make 1000 mL of total volume. It was adjusted to a pH of 6.45 ∼ 6.55 by adding sodium hydroxide or phosphoric acid.
   - Mobile phase: Disodium hydrogen phosphate anhydrous 1.42 g was dissolved in water 1000 mL, and then adjusted to a pH of 6.0 by adding diluted phosphoric acid. After that, sodium 1-octanesulfonate 4.32 g was added. 500 mL of a mixed solution of acetonitrile and methanol (900:100) was added to 500 mL of the solution obtained, and then filtered.
   - Diluent: a mixed solution of methanol, acetonitrile and water (5:55:40)
   - Flow rate: 1.0 mL/min
   - Measurement time of peak areas: 10 min

**[Table 9]**

| | Dissolution rate of candesartan after 15 minutes (%) | | |
|---|---|---|---|
| | Average | Min. | Max. |
| Ex. 1 | 78.4 | 76.2 | 80.8 |
| Ex. 2 | 80.1 | 76.5 | 83.1 |
| Ex. 3 | 81.0 | 77.1 | 82.1 |
| Ex. 4 | 80.8 | 78.2 | 82.9 |
| Ex. 5 | 75.0 | 72.7 | 78.6 |
| Ex. 6 | 79.1 | 75.4 | 81.2 |
| Comp. Ex. 1 | 68.4 | 67.1 | 69.2 |
| Comp. Ex. 2 | 58.0 | 55.2 | 59.8 |
| Comp. Ex. 3 | 60.8 | 55.6 | 65.0 |
| Comp. Ex. 4 | 56.0 | 49.1 | 66.2 |
| Comp. Ex. 5 | 35.2 | 30.7 | 37.7 |

As shown in Table 9, the dissolution rate of candesartan active ingredient remarkably increased when macrogol-15-hydroxystearate was used as a solubilizer in comparison with when different types of solubilizers were used at the same amount or when any solubilizer was not used.

It means that although the water-solubility and bioavailability of the active ingredient candesartan is low, its property dissolved from the tablet in the GI tract is improved in the tablet according to the present invention. Accordingly, the tablet makes the level of drug in the blood to be maintained at the constant level, and the treatment effect of hypertension to be achieved at the desired level.

### Experimental Example 4

The impact of sugar alcohol(s) in a tablet was evaluated by assessing the dissolution rate, hardness, tabletability, and productivity of tablet.

The tablets of Comparative Example 6 to 11 were prepared by the same method as Examples 1 to 6, except that mannitol is comprised as sugar alcohol. In order to minimize an experimental error by making equal both total weights of Comparative Examples 6 to 11 and of the corresponding Examples 1 to 6, the amount of microcrystalline cellulose or microcrystalline cellulose + silicified microcrystalline cellulose was reduced by the amount of mannitol further added in Comparative Example 6 to 11.

Table 10 below shows the composition and weight of tablets prepared in Comparative Examples 6 to 11. In Table 10, "Comp. Ex." stands for "Comparative Example". The product prepared in the Comp. Ex. 6, 7, 8, 9 and 11 respectively is a double-layer tablet, whereas the product prepared in the Comp. Ex. 10 is a single-layer tablet. In Table 10, "the extragranular mixing-part" indicates the part formed on the outside of granule by mixing additional materials following granulation.

**[Table 10]**

| Composition | Weight (g) | | | | | |
|---|---|---|---|---|---|---|
| | Comp. Ex. 6 | Comp. Ex. 7 | Comp. Ex. 8 | Comp. Ex. 9 | Comp. Ex. 10 | Comp. Ex. 11 |
| | | | | | | |
| **The granule-part in the first layer of double-layer tablet** | | | | | | |
| candesartan cilexetil | 16.00 | 16.00 | 8.00 | 8.00 | - | 16.00 |
| microcrystalline cellulose | 13.00 | 13.00 | 6.50 | 6.50 | - | 13.00 |
| pregelatinized starch | 10.00 | 10.00 | 5.00 | 5.00 | - | 10.00 |
| hydroxypropylcellulose | 6.00 | 6.00 | 3.00 | 3.00 | - | 6.00 |
| macrogol-15-hydroxystearate | 3.00 | 3.00 | 1.50 | 1.50 | - | 3.00 |
| silicified microcrystalline cellulose | - | - | - | - | - | 16.00 |
| mannitol | 52.00 | 52.00 | 26.00 | 26.00 | - | 115.00 |
| | | | | | | |

| **The extragranular mixing-part in the first layer of double-layer tablet** | | | | | | |
|---|---|---|---|---|---|---|
| silicified microcrystalline cellulose | 16.00 | 16.00 | 8.00 | 8.00 | - | - |
| magnesium stearate | 1.00 | 1.00 | 0.50 | 0.50 | - | 1.00 |
| mannitol | 63.00 | 63.00 | 31.50 | 31.50 | - | - |
| | | | | | | |

| **The granule-part in the second layer of double-laver tablet** | | | | | | |
|---|---|---|---|---|---|---|
| amlodipine besylate | 13.87 | 6.935 | 6.935 | 3.468 | - | 13.87 |
| microcrystalline cellulose | 14.00 | 7.00 | 7.00 | 8.00 | - | 27.00 |
| pregelatinized starch | 10.00 | 5.00 | 5.00 | 5.00 | - | 25.00 |
| hydroxypropylcellulose | 5.00 | 2.50 | 2.50 | 2.50 | - | 5.00 |
| mannitol | 57.13 | 28.565 | 28.565 | 31.032 | - | 108.13 |
| | | | | | | |

| **The extragranular mixing-part in the second layer of double-layer tablet** | | | | | | |
|---|---|---|---|---|---|---|
| microcrystalline cellulose | 13.00 | 6.50 | 6.50 | 6.50 | - | - |
| pregelatinized starch | 15.00 | 7.50 | 7.50 | 7.50 | - | - |
| magnesium stearate | 1.00 | 0.50 | 0.50 | 0.50 | - | 1.00 |
| mannitol | 51.00 | 25.50 | 25.50 | 25.50 | - | - |
| | | | | | | |

| **The first-granule-part in the single-layer tablet** | | | | | | |
|---|---|---|---|---|---|---|
| candesartan cilexetil | - | - | - | - | 16.00 | - |
| microcrystalline cellulose | - | - | - | - | 13.00 | - |
| pregelatinized starch | - | - | - | - | 10.00 | - |
| hydroxypropylcellulose | - | - | - | - | 6.00 | - |
| macrogol-15-hydroxystearate | - | - | - | - | 3.00 | - |
| mannitol | - | - | - | - | 52.00 | - |
| | | | | | | |

| **The second-granule-part in the single-layer tablet** | | | | | | |
|---|---|---|---|---|---|---|
| amlodipine besylate | - | - | - | - | 13.87 | - |
| microcrystalline cellulose | - | - | - | - | 14.00 | - |
| pregelatinized starch | - | - | - | - | 10.00 | - |
| hydroxypropylcellulose | - | - | - | - | 5.00 | - |
| mannitol | - | - | - | - | 57.13 | - |
| | | | | | | |

| **The extragranular mixing-part in the single-layer tablet** | | | | | | |
|---|---|---|---|---|---|---|
| microcrystalline cellulose | - | - | - | - | 13.00 | - |
| silicified microcrystalline cellulose | - | - | - | - | 16.00 | - |
| pregelatinized starch | - | - | - | - | 15.00 | - |
| magnesium stearate | - | - | - | - | 2.00 | - |
| mannitol | - | - | - | - | 114.00 | - |
| | | | | | | |

| **The coating-part** | | | | | | |
|---|---|---|---|---|---|---|
| OPADRY | 10.00 | 7.50 | 5.00 | 5.00 | 10.00 | 10.00 |
| | | | | | | |
| **Total weight** | 370.0 | 277.5 | 185.0 | 185.0 | 370.0 | 370.0 |

The dissolution rates of candesartan active ingredient were determined as in Experimental Example 3 on the coated tablets prepared in Examples 1 to 6 and Comparative Examples 6 to 11. Six tablets of Examples and Comparative Examples were tested respectively, and Table 11 below shows the average value, minimum value and maximum value of dissolution rate (%).

**[Table 11]**

| Dissolution rate of candesartan after 15 minutes (%) | | | | | | | |
|---|---|---|---|---|---|---|---|
| | Average | Min. | Max. | | Average | Min. | Max. |
| Ex. 1 | 78.4 | 76.2 | 80.8 | Comp. Ex. 6 | 59.7 | 55.0 | 63.9 |
| Ex. 2 | 80.1 | 76.5 | 83.1 | Comp. Ex. 7 | 52.6 | 48.7 | 54.6 |
| Ex. 3 | 81.0 | 77.1 | 82.1 | Comp. Ex. 8 | 60.8 | 55.8 | 65.4 |
| Ex. 4 | 80.8 | 78.2 | 82.9 | Comp. Ex. 9 | 51.0 | 47.7 | 53.3 |
| Ex. 5 | 75.0 | 72.7 | 78.6 | Comp. Ex. 10 | 50.7 | 48.6 | 53.0 |
| Ex. 6 | 79.1 | 75.4 | 81.2 | Comp. Ex. 11 | 50.6 | 47.9 | 53.1 |

As shown in Table 11, the dissolution rate of candesartan active ingredient in the tablet comprising sugar alcohol such as mannitol (Comparative Examples 6 to 11) was about 20 ∼ 30% lower than that in the tablet not comprising any sugar alcohol (Examples 1 to 6).

Next, the hardness was measured on the coated tablets prepared in Examples 1 to 6 and Comparative Examples 6 to 11 respectively, and the results are shown in Table 12.

**[Table 12]**

| Hardness | | | |
|---|---|---|---|
| Ex. 1 | 18 ∼ 19 kp | Comp. Ex. 6 | 12 ∼ 13 kp |
| Ex. 2 | 16 ∼ 17 kp | Comp. Ex. 7 | 9 ∼ 10 kp |
| Ex. 3 | 15 ∼ 16 kp | Comp. Ex. 8 | 10 ∼ 11 kp |
| Ex. 4 | 16 ∼ 17 kp | Comp. Ex. 9 | 9 ∼ 10 kp |
| Ex. 5 | 15 ∼ 16 kp | Comp. Ex. 10 | 12 ∼ 13 kp |
| Ex. 6 | 16 ∼ 17 kp | Comp. Ex. 11 | 12 ∼ 13 kp |

As shown in Table 12, the hardness of tablets comprising sugar alcohol such as mannitol (Comparative Examples 6 to 11) was about 20 ∼ 40% lower than that of tablets not comprising any sugar alcohol (Examples 1 to 6). If it does not have a sufficient hardness, thefriability and the possibility of coating defect increase in the manufacturing process thereof, particularly in the coating process thereof. It means that the uniform quality of tablets cannot be assured, and the productivity is not good.

Therefore, if a sugar alcohol is comprised in the tablet of the present invention using macrogol-15-hydroxystearate as a solubilizer, there is a decrease in the dissolution rate of active ingredient candesartan or candesartan cilexetil and the hardness of tablet, and thereby it is disadvantageous in terms of tabletability and productivity. Accordingly, it is preferable that any sugar alcohol is not comprised in the tablet of the present invention.

### Experimental Example 5: Safety study

A study was conducted to evaluate the safety in human for the combination formulation of the present invention.

A combination formulation of the present invention was prepared comprising candesartan cilexetil 16 mg and amlodipine 10 mg and comprising macrogol-15-hydroxystearate as a solubilizer. A single dose of tablet obtained was orally administered to 46 healthy adult males at the age of 19 ∼ 45.

As a result, it was observed that adverse events such as dizziness occurred in some subjects, but there was no serious adverse drug reaction which was clinically significant. It means that the combination formulation according to the present invention is safe for human beings.

## Claims

1. A tablet comprising candesartan or candesartan cilexetil as the first active ingredient and amlodipine or its pharmaceutically acceptable salt as the second active ingredient, **characterized in that** it comprises macrogol-15-hydroxystearate as a solubilizer.

2. The tablet of claim 1, wherein the first active ingredient is candesartan cilexetil.

3. The tablet of claim 1, wherein it does not comprise any sugar alcohol.

4. The tablet of claim 1, wherein the weight ratio of candesartan or candesartan cilexetil to macrogol-15-hydroxystearate is 1:0.03 ∼ 1:2.

5. The tablet of claim 1, wherein it is single-layer tablet, multi-layer tablet or nucleated tablet.

6. The tablet of claim 5, wherein the multi-layer tablet is double-layer tablet.

7. The tablet of claim 6, wherein the double-layer tablet is composed of the first layer and the second layer, the first layer comprising candesartan or candesartan cilexetil and macrogol-15-hydroxystearate, and the second layer comprising amlodipine or its pharmaceutically acceptable salt.

8. The tablet of claim 1, wherein its storage period is 6 weeks to 3 years.

## Patentansprüche

1. Eine Tablette, umfassend Candesartan oder Candesartancilexetil als den ersten aktiven Bestandteil und Amlodipin oder sein pharmazeutisch verträgliches Salz als den zweiten aktiven Bestandteil, **dadurch gekennzeichnet, dass** sie Macrogol-15-hydroxystearat als einen Lösungsvermittler umfasst.

2. Die Tablette nach Anspruch 1, wobei der erste aktive Bestandteil Candesartancilexetil ist.

3. Die Tablette nach Anspruch 1, wobei sie keinen Zuckeralkohol umfasst.

4. Die Tablette nach Anspruch 1, wobei das Gewichtsverhältnis von Candesartan oder Candesartancilexetil zu Macrogol-15-hydroxystearat 1:0,03 bis 1:2 beträgt.

5. Die Tablette nach Anspruch 1, wobei sie eine einschichtige Tablette, mehrschichtige Tablette oder nukleierte Tablette ist.

6. Die Tablette nach Anspruch 5, wobei die mehrschichtige Tablette eine zweischichtige Tablette ist.

7. Die Tablette nach Anspruch 6, wobei die zweischichtige Tablette aus der ersten Schicht und der zweiten Schicht besteht, wobei die erste Schicht Candesartan oder Candesartancilexetil und Macrogol-15-hydroxystearat umfasst und die zweite Schicht Amlodipin oder sein pharmazeutisch verträgliches Salz umfasst.

8. Die Tablette nach Anspruch 1, wobei die Lagerzeit 6 Wochen bis 3 Jahre beträgt.

## Revendications

1. Comprimé comprenant du candésartan ou du candésartan cilexétil en tant que premier principe actif et de l'amlodipine ou son sel pharmaceutiquement acceptable en tant que deuxième principe actif, **caractérisé en ce qu'**il comprend du 15-hydroxystéarate de macrogol en tant que solubilisant.

2. Comprimé selon la revendication 1, dans lequel le premier principe actif est le candésartan cilexétil.

3. Comprimé selon la revendication 1, qui ne comprend aucun alcool de sucre.

4. Comprimé selon la revendication 1, dans lequel le rapport en poids du candésartan ou du candésartan cilexétil au 15-hydroxystéarate de macrogol est de 1/0,03 à 1/2.

5. Comprimé selon la revendication 1, qui est un comprimé monocouche, un comprimé multicouche ou un comprimé nucléé.

6. Comprimé selon la revendication 5, dans lequel le comprimé multicouche est un comprimé double couche.

7. Comprimé selon la revendication 6, dans lequel le comprimé double couche est composé d'une première couche et d'une deuxième couche, la première couche comprenant du candésartan ou du candésartan cilexétil et du 15-hydroxystéarate de macrogol, et la deuxième couche comprenant de l'amlodipine ou son sel pharmaceutiquement acceptable.

8. Comprimé selon la revendication 1, dans lequel sa période de stockage est de 6 semaines à 3 ans.
